# EUROPEAN PATENT APPLICATION

(11) **EP 2 163 200 A1**
(43) Date of publication of application: **17.03.2010**
(21) Application number: 08721939.0
(22) Date of filing: 12.03.2008
(51) Int. Cl.: A61B 5/157, A61B 5/1473, A61B 5/1486, A61B 5/151

(54) **BODY FLUID COLLECTING CIRCUIT BOARD**

(30) Priority: 11.07.2007 JP 2007182404
(71) Applicant: Nitto Denko Corporation, Ibaraki-shi, Osaka 567-8680 (JP)
(72) Inventor: KANETO, Masayuki, Ibaraki-shi Osaka 567-8680 (JP); IWASAKI, Hirokazu, Ibaraki-shi Osaka 567-8680 (JP); ISHII, Jun, Ibaraki-shi Osaka 567-8680 (JP); NAITO, Toshiki, Ibaraki-shi Osaka 567-8680 (JP)
(74) Representative: Schwabe - Sandmair - Marx
(86) International application number: PCT/JP2008/054523
(87) International publication number: WO 2009/008193

(57) **Abstract**

A body fluid collecting circuit board is provided with an insulating layer, a puncture needle supported by the insulating layer and a conductor pattern that integrally has an electrode which is supported by the insulating layer and brings body fluid collected by puncture of the puncture needle into contact with the board, a terminal connected to a device measuring a component of body fluid and wiring for electrically connecting the electrode and the terminal.

## Description

### Technical Field

The present invention relates to a circuit board for body fluid collection, and particularly to a circuit board for body fluid collection which is connected to a device for measuring a component of a body fluid, and used to measure a component of a body fluid.

### Background Art

Diabetes mellitus includes insulin-dependent (type I) diabetes and non-insulin-dependent (type II) diabetes. The former type of diabetes necessitates regular administration of insulin. Therefore, for a patient with the former type of diabetes, a treatment method has been employed in which the patient measures his or her blood sugar value, and administers insulin to himself or herself at a dosage in accordance with the blood sugar value.
A blood-sugar-value measuring device which allows a patient to personally collect his or her blood oneself, and measure his or her blood sugar value has been known solely to such a patient.

For example, there has been proposed a fluid collecting device which integrally includes a puncture needle, a reaction zone into which electrodes are inserted, and a capillary channel connecting the puncture needle and the electrodes to each other (see, e.g., Patent Document 1).
For example, there has been also proposed a sensor in which contact portions for contact with blood, terminal portions for connecting to a blood-sugar measuring device, and wires connecting the contact portions and the terminal portions are formed in the same base material for sensor with printed wiring.
Patent Document 1: Japanese Unexamined Patent No. 2004-493
Patent Document 2: Japanese Unexamined Patent No. 2006-15068

### Disclosure of the Invention

### Problems to be Solved

In the fluid collecting device described in Patent Document 1, the puncture needle and the reaction zone are integrally formed, so that preparations for measurement are easy. However, in the fluid collecting device, the electrodes which are members separate from the reaction zone are inserted into the reaction zone to measure a blood component. This leads to a problem that the accuracy of sensing blood is unstable, and accurate measurement cannot be performed.
On the other hand, in the sensor described in Patent Document 2, the contact portions, the terminal portions, and the wires are formed on the same base for sensor with the printed wiring, so that the accuracy of sensing blood is stable. However, since the sensor is not provided with a puncture needle, when measurement is performed with the sensor, it is necessary to first cause bleeding with a puncture needle provided in the blood sugar measuring device, and then collect blood into the sensor, which results in the problem of an intricate operation.

An object of the present invention is to provide a circuit board for body fluid collection which allows accurate measurement of a component of a body fluid with a simple structure, and is easily operated.

### Means for Solving the Problems

To attain the object, a circuit board for body fluid collection of the present invention includes an insulating layer, a puncture needle supported on the insulating layer, and a conductive pattern supported on the insulating layer, and integrally including an electrode to be brought into contact with a body fluid collected by puncture with the puncture needle, a terminal to be connected to a device for measuring a component of the body fluid, and a wire electrically connecting the electrode and the terminal.

In the circuit board for body fluid collection, the puncture needle and the conductive pattern are integrally supported on the insulating layer. Accordingly, it is possible to cause the body fluid to flow out by puncture with the puncture needle, and easily bring the flown-out body fluid into contact with the electrode. Additionally, in the circuit board for body fluid collection, the electrode, the terminal, and the wire are provided integrally as the conductive pattern. Therefore, it is possible to improve the accuracy of sensing a component of the body fluid in contact with the electrode, and improve measurement accuracy. As a result, the circuit board for body fluid collection allows accurate measurement of a component of the body fluid with a simple structure, and allows easy operation.

In the circuit board for body fluid collection of the present invention, it is preferable that a stopper for restricting further puncture with the puncture needle is provided on an upstream side of the puncture needle in a puncture direction thereof. When the stopper is provided, puncture with the puncture needle is restricted by the stopper. This can ensure reliable and stable puncture.
It is further preferable that the stopper is provided at a position spaced apart from a tip of the puncture needle by 0.3 to 2.0 mm in the puncture direction of the puncture needle. When the position of the stopper is in the range shown above, it is possible to reliably prevent excessive puncture with the puncture needle.

In the circuit board for body fluid collection of the present invention, it is preferable that the insulating layer is provided with a dam that is disposed around the electrode in order to prevent leakage of the body fluid from the electrode. When the dam is provided, it is possible to prevent leakage of the body fluid from the electrode with the dam. This allows the achievement of accurate measurement of a component of the body fluid.

### Effect of the Invention

The circuit board for body fluid collection allows accurate measurement of a component of a body fluid with a simple structure, and allows easy operation.

### Brief Description of the Drawings

[FIG. 1] FIG. 1 shows a circuit board for blood collection as an embodiment of a circuit board for body fluid collection of the present invention,
   (a) showing a plan view thereof, and
   (b) showing a longitudinal cross-sectional view (cross-sectional view along the line A-A of (a)) thereof.
[FIG. 2] FIG 2 is an example of a production process view (cross-sectional view along the line B-B of FIG. 1(a)) of the circuit board for blood collection shown in FIG. 1,
   (a) showing the step of preparing a metal board,
   (b) showing the step of forming an insulating base layer,
   (c) showing the step of forming a conductive pattern,
   (d) showing the step of forming an insulating cover layer,
   (e) showing the step of trimming the metal board, and
   (f) showing the step of coating an electrode with a chemical agent.

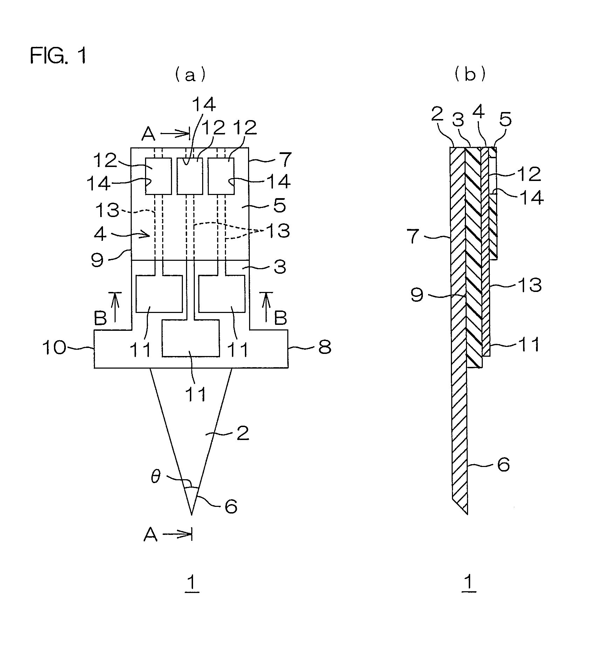

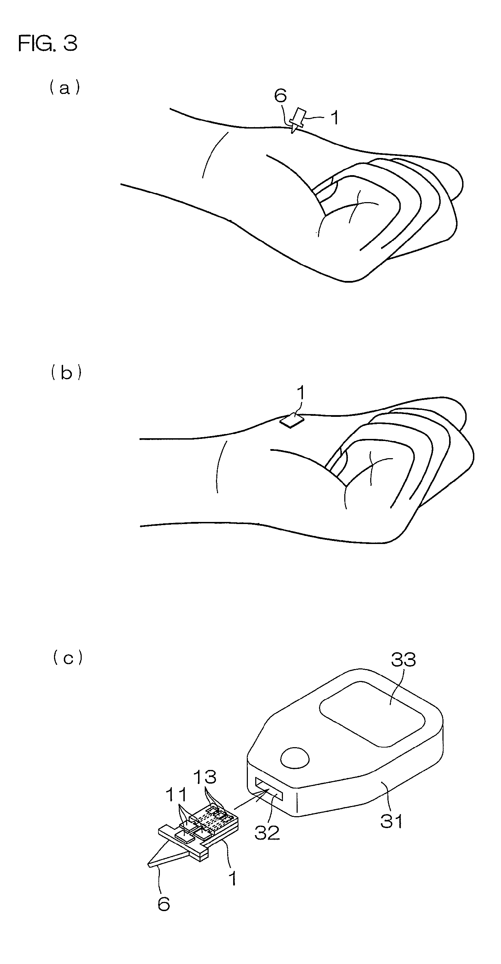

[FIG. 3] FIG. 3 is an illustrative view showing an example of a method of using the circuit board for blood collection shown in FIG. 1,
   (a) showing a state where a puncture needle punctures a portion to be punctured,
   (b) showing a state where electrodes are brought into contact with the punctured portion, and
   (c) showing a state where the circuit board for blood collection is inserted into a blood-sugar-value measuring device.

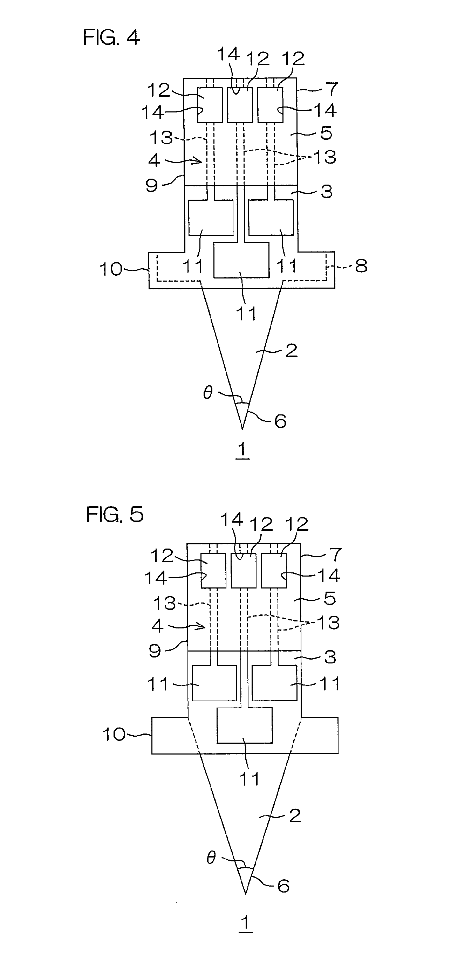

[FIG. 4] FIG. 4 is a plan view showing a circuit board for blood collection as an embodiment (implementation in which the downstream-side end edge of an insulating-side stopper in a puncture direction is disposed downstream in the puncture direction from the downstream-side end edge of a board-side stopper in the puncture direction) of the circuit board for body fluid collection of the present embodiment.
[FIG. 5] FIG. 5 is a plan view showing a circuit board for blood collection as an embodiment (implementation in which only the insulating-side stopper is formed) of the circuit board for body fluid collection of the present invention.

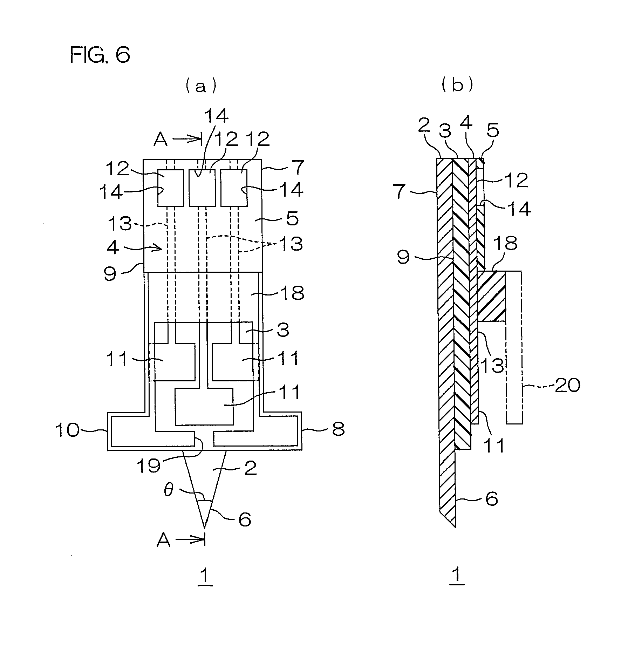

[FIG. 6] FIG. 6 shows a circuit board for blood collection as an embodiment (implementation in which a dam is formed) of the circuit board for body fluid collection of the present invention,
   (a) showing a plan view thereof, and
   (b) showing a longitudinal cross-sectional view (cross-sectional view along the line A-A of (a)) thereof.

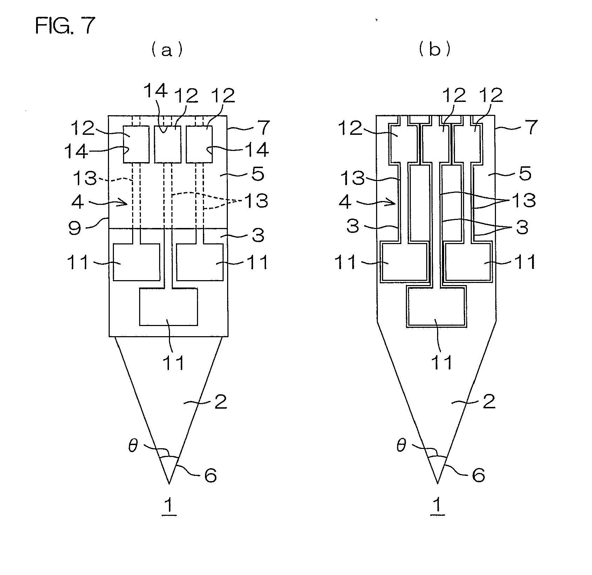

[FIG. 7] FIG. 7 is a plan view showing a circuit board for blood collection as an embodiment (implementation in which the insulating-side stopper and the board-side stopper are not formed) of the circuit board for body fluid collection of the present invention,
   (a) showing an implementation including the insulating base layer and the insulating cover layer, and
   (b) showing an implementation including only the insulating base layer.

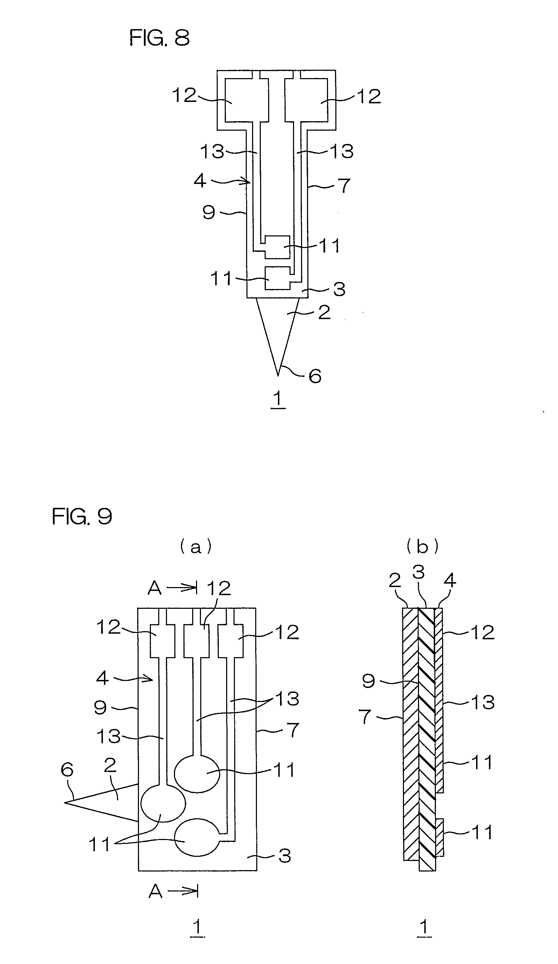

[FIG. 8] FIG. 8 is a plan view showing a circuit board for blood collection as an embodiment (implementation in which two electrodes are formed) of the circuit board for body fluid collection of the present invention.
[FIG. 9] FIG. 9 shows a circuit board for blood collection as an embodiment (implementation in which the puncture needle is provided along the widthwise direction of the circuit board for blood collection) of the circuit board for body fluid collection of the present invention,
   (a) showing a plan view thereof, and
   (b) showing a longitudinal cross-sectional view (cross-sectional view along the line A-A of (a)) thereof.

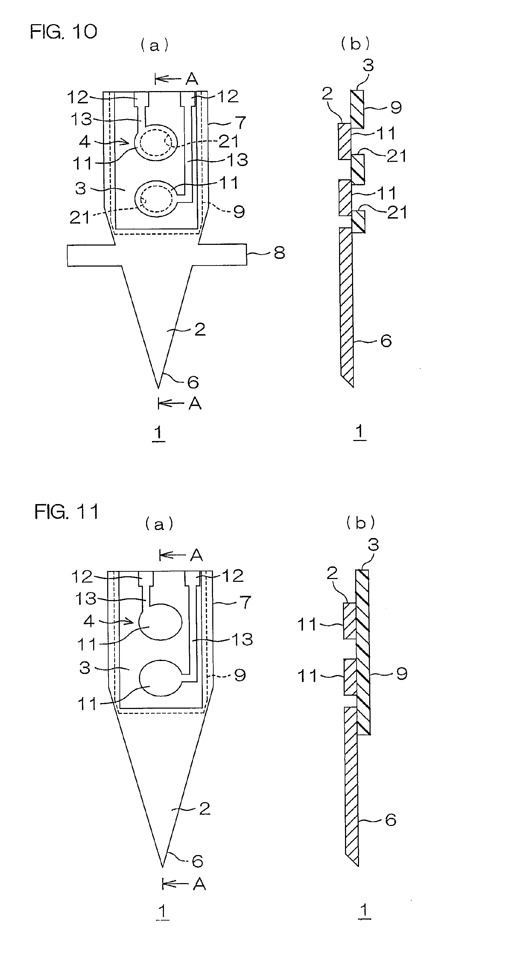

[FIG. 10] FIG. 10 shows a circuit board for blood collection as an embodiment (implementation in which the conductive pattern with an opening is formed from the metal board) of the circuit board for body fluid collection of the present invention,
   (a) showing a rear view thereof, and
   (b) showing a longitudinal cross-sectional view (cross-sectional view along the line A-A of (a)) thereof.
[FIG. 11] FIG. 11 shows a circuit board for blood collection as an embodiment (implementation in which the conductive pattern without an opening is formed from the metal board) of the circuit board for body fluid collection of the present invention,
   (a) showing a rear view thereof, and
   (b) showing a longitudinal cross-sectional view (cross-sectional view along the line A-A of (a)) thereof.

### Embodiments of the Invention

FIG. 1 shows a circuit board for blood collection as an embodiment of a circuit board for body fluid collection of the present invention, (a) showing a plan view thereof, and (b) showing a longitudinal cross-sectional view thereof.
In FIG. 1, a circuit board for blood collection 1 is used in combination with a blood-sugar-value measuring device 31 (see FIG. 3) for a patient to puncture the skin of his or her finger or the like, and measure an amount of glucose in blood. The circuit board for blood collection 1 is prepared as a disposable type to be disposed of after each measurement.

As shown in FIG 1(b), the circuit board for blood collection 1 includes a metal board 2, an insulating base layer 3 as an insulating layer laminated on the surface of the metal board 2, a conductive pattern 4 laminated on the surface of the insulating base layer 3, and an insulating cover layer 5 provided on the surface of the insulating base layer 3 so as to cover the conductive pattern 4.
The metal board 2 is formed of a metal foil or the like in a rectangular shape extending in a longitudinal direction. Examples of a metal used to form the metal board 2 include nickel, chromium, iron, and stainless steel (SUS304, SUS430, or SUS316L). Preferably, stainless steel is used. The thickness of the metal board 2 is in a range of, e.g., 10 to 300 µm, or preferably 20 to 100 µm. When the thickness thereof is less than 10 µm, skin may not be able to be punctured due to an insufficient strength. On the other hand, when the thickness thereof is in excess of 300 µm, skin may be excessively damaged with feeling pain upon puncture.

As shown in FIG. 1(a), the metal board 2 integrally includes a puncture needle 6, a circuit board portion 7, and a board-side stopper 8 as a stopper.
The puncture needle 6 is formed in a generally triangular plan view shape (in an isosceles triangular shape) having a tip pointed at an acute angle along the longitudinal direction. The angle θ of the tip thereof is in a range of, e.g., 10 to 30°, or preferably 15 to 25°. When the angle θ of the tip is less than 10°, skin may not be able to be punctured due to an insufficient strength. On the other hand, when the angle θ thereof is in excess of 30°, skin may be hard to puncture. The longitudinal length of the puncture needle 6 is in a range of, e.g., 0.3 to 2 mm.

The circuit board portion 7 is disposed on an upstream side of the puncture needle 6 in a puncture direction thereof, and provided continuously from the puncture needle 6. The circuit board portion 7 is formed in a generally rectangular plan view shape (in an oblong rectangular plan view shape) which is oblong in the puncture direction. The length of the circuit board portion 7 in a widthwise direction (direction perpendicular to the longitudinal direction) is in a range of, e.g., 50 µm to 30 mm. The longitudinal length of the circuit board portion 7 is in a range of, e.g., 2 to 25 mm. The longitudinal length of the metal board 2 (the longitudinal lengths of the puncture needle 6 and the circuit board portion 7) is in a range of, e.g., 5 to 30 mm.

The board-side stopper 8 is provided at the upstream-side end portion of the puncture needle 6 in the puncture direction thereof. The board-side stopper 8 is formed at the upstream-side end portion of the puncture needle 6 in the puncture direction thereof so as to protrude from the both widthwise sides of the upstream-side end portion along the widthwise direction. The widthwise protruding length of the board-side stopper 8 is in a range of, e.g., 0.1 to 2 mm. The board-side stopper 8 is formed in a generally rectangular plan view shape so as to be wider than the circuit board portion 7, and the downstream-side end edge thereof in the puncture direction is formed in a straight line in the widthwise direction. The downstream-side end edge of the board-side stopper 8 in the puncture direction is disposed to be spaced apart from the tip of the puncture needle 6 toward the upstream side of the puncture needle 6 in the puncture direction thereof by 0.3 to 2.0 mm. When the position of the board-side stopper 8 is within the range shown above, it is possible to reliably prevent excessive puncture with the puncture needle 6.

The insulating base layer 3 integrally includes a circuit mounting portion 9 laminated on the surface of the circuit board portion 7, and an insulating-side stopper 10 as a stopper laminated on the surface of the board-side stopper 8. The circuit mounting portion 9 is formed in the same shape as that of the circuit board portion 7 when viewed in plan view. The insulating-side stopper 10 is formed in the same shape as the board-side stopper 8 when viewed in plan view.
Examples of an insulating material used to form the insulating base layer 3 include synthetic resins such as a polyimide resin, a polycarbonate resin, a polyethylene resin, a polyethylene terephthalate resin, an epoxy resin, and a fluorine resin. In terms of mechanical endurance and resistance to a chemical agent, a polyimide resin is preferably used. The thickness of the insulating base layer 3 is in a range of, e.g., 3 to 50 µm, or preferably 5 to 25 µm. When the thickness thereof is less than 3 µm, an insulation defect such as a pinhole may occur. On the other hand, when the thickness thereof is in excess of 50 µm, cutting and trimming may be hard to perform.

The conductive pattern 4 includes three electrodes 11, three terminals 12, and three wires 13.
The three electrodes 11 are disposed on the downstream-side portion of the circuit mounting portion 9 in the puncture direction. These electrodes 11 are each formed in a generally rectangular plan view shape, of which the two are arranged in parallel in the widthwise direction, and the remaining one is disposed on the downstream side of the foregoing two in the puncture direction. The three electrodes 11 respectively correspond to a working electrode, a counter electrode, and a reference electrode. The length of one side of each of the electrodes 11 is in a range of, e.g., 100 µm to 2.5 mm. The three electrodes 11 are each disposed within a range of, e.g., 0.2 to 5 mm, or preferably 0.5 to 3 mm from the tip of the puncture needle 6 in the puncture direction. When the distances between the tip of the puncture needle 6 and the electrodes 11 are excessively short, the electrodes 11 may pierce skin together with the puncture needle 6, and a chemical agent 17 (described later) coated on the surfaces of the electrodes 11 may be dispersed into a body to inhibit precise measurement. On the other hand, when the distances between the tip of the puncture needle 6 and the electrodes 11 are excessively long, a structure for using suction and capillarity is needed to introduce blood from the puncture needle 6 into the electrodes 11.

The three terminals 12 are provided in opposed relation to the three electrodes 11, and disposed on the upstream-side portion of the circuit mounting portion 9 in the puncture direction. These terminals 12 are each formed in a generally rectangular plan view shape slightly smaller than each of the electrodes 11. The three terminals 12 are arranged in parallel in the widthwise direction.
The three wires 13 are arranged in parallel and mutually spaced-apart relation in the widthwise direction, and provided along the longitudinal direction so as to electrically connect the respective electrodes 11 and the corresponding terminals 12. Each of the electrodes 11, the terminal 12 opposed thereto, and the wire 13 connected to the electrode 11 and the terminal 12 are provided continuously and integrally. The widthwise length of each of the wires 13 is in a range of, e.g., 0.01 to 2 mm. The longitudinal length of each of the wires 13 is in a range of, e.g., 5 to 28 mm.

Examples of a conductive material used to form the conductive pattern 4 include metals such as iron, nickel, chromium, copper, gold, silver, platinum, and an alloy thereof. An appropriate conductive material is selected in terms of adhesion to the insulating base layer 3 and the insulating cover layer 5 and easy processing. It is also possible to laminate two or more kinds of conductive materials.
The insulating cover layer 5 is provided on the surface of the circuit mounting portion 9 so as to cover each of the wires 13. Specifically, the downstream-side end edge of the insulating cover layer 5 in the puncture direction is formed in a straight line along the widthwise direction on the upstream side of the three electrodes 3 in the puncture direction so as to expose the three electrodes 11. The insulating cover layer 5 is formed with openings 14 for exposing the respective terminals 12. As an insulating material for forming the insulating cover layer 5, the same insulating material as that of the insulating base layer 3 shown above is used. The thickness of the insulating cover layer 5 is in a range of, e.g., 2 to 50 µm.

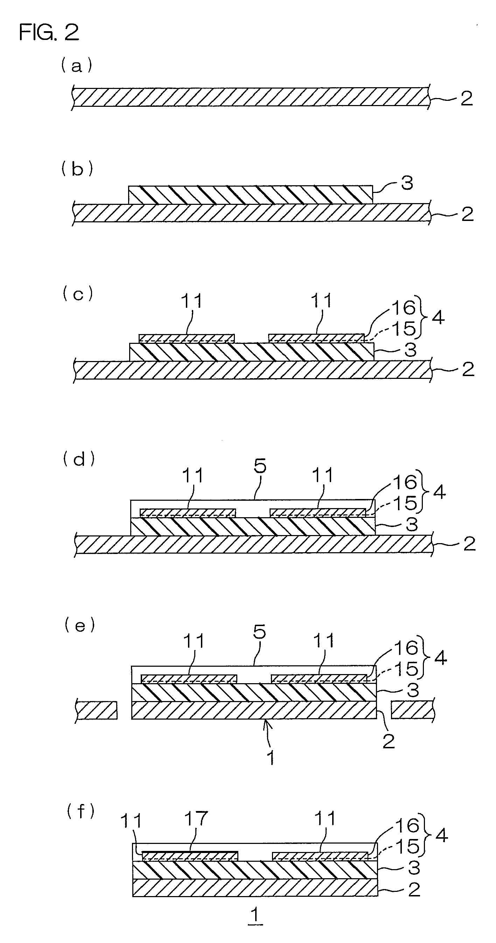

FIG. 2 is a production process view showing an example of a producing method of the circuit board for blood collection 1. Next, referring to FIG. 2, a description is given to the producing method of the circuit board for blood collection 1.
In this method, as shown in FIG. 2(a), the metal board 2 is prepared first. The metal board 2 is prepared as, e.g., an elongated metal foil which allows a large number of the metal boards 2 to be secured therefrom. From the elongated metal foil, a plurality of the circuit boards for blood collection 1 are produced by trimming (described later) the respective metal boards 2.

Next in this method, as shown in FIG. 2(b), the insulating base layer 3 is formed on the surface of the metal board 2. For the formation of the insulating base layer 3, there is used a method in which, e.g., a varnish of a photosensitive synthetic resin is coated on the surface of the metal board 2, and cured after photoprocessing, a method in which, e.g., a film of a synthetic resin is laminated on the surface of the metal board 2, an etching resist in the same pattern as that of the insulating base layer 3 is laminated on the surface of the film, and then the film exposed from the etching resist is wet-etched, a method in which, e.g., a film of a synthetic resin mechanically punched in advance is laminated on the surface of the metal board 2, a method in which, e.g., a film of a synthetic resin is laminated on the surface of the metal board 2, and then subjected to discharge processing or laser processing, or the like. In terms of processing accuracy, the method in which a varnish of a photosensitive synthetic resin is coated on the surface of the metal board 2, and then cured after photoprocessing is preferably used.

Thereafter in this method, as shown in FIG. 2(c), the conductive pattern 4 is formed. For the formation of the conductive pattern 4, there is used a known patterning method in which printed wiring is formed, such as an additive method or a subtractive method. In terms of enabling the formation of a minute pattern, the additive method is preferably used. In the additive method, for example, a metal thin film 15 is formed on the surface of the insulating base layer 3 by chemical vapor deposition or sputtering, a plating resist is formed on the surface of the metal thin film 15, and then a plating layer 16 is formed on the surface of the metal thin film 15 exposed from the plating resist by electrolytic plating using the metal thin film 15 as a seed film.

The conductive pattern 4 can also be formed only from the metal thin film 15 by chemical vapor deposition or sputtering.
Note that, in the formation of the conductive pattern 4, it is also possible to further form a plating layer of a different metal on the surface of each of the electrodes 11 and on the surface of each of the terminals 12 by electrolytic plating or electroless plating.
Next in this method, as shown in FIG. 2(d), the insulating cover layer 5 is formed. For the formation of the insulating cover layer 5, the same method as the method used to form the insulating layer 3 is used. Preferably, a method is used in which a varnish of a photosensitive synthetic resin is coated on the surface of the insulating base layer 3 so as to cover the conductive pattern 4, and then cured after photoprocessing. Note that, in the case of forming the insulating cover layer 5 having the openings 14 in a pattern, the insulating cover layer 5 may be formed appropriately in a pattern having the openings 14. The openings 14 can also be bored by a method in which, e.g., discharge processing is performed, a method in which, e.g., laser processing is performed, or the like.

Thereafter, as shown in FIG. 2(e), the metal board 2 is trimmed. For the trimming of the metal board 2, there is used, e.g., discharge processing, laser processing, mechanical punching processing, etching processing, or the like. In terms of easy cleaning after processing, etching processing (wet etching) is preferably used.
In this manner, the circuit board for blood collection 1 in which the puncture needle 6 and the conductive pattern 4 are supported on the insulating base layer 3 can be obtained. In the obtained circuit board for blood collection 1, as shown in FIG. 2(f), the chemical agent 17 is coated, i.e., e.g., glucose oxidase, glucose dehydrogenase, or the like as an enzyme and, e.g., potassium ferricyanide, ferrocene, benzoquinone, or the like as a mediator are coated alone or in combination on each of the electrodes 11. For the coating of the chemical agent 17, there is used an appropriate method such as, e.g., a dipping method, a spray method, or an inkjet method.

Depending on the type of the agent 17, after the plating layer of a different metal is formed on the surface of the electrode 11 as described above, it is also possible to further form a coating of a different metal in advance, and provide a predetermined potential difference therebetween. Specifically, it is shown by way of example to form a gold plating layer, and then further coat silver or a silver chloride on the surface of the gold plating layer.
FIG. 3 is an illustrative view showing an example of a method of using the circuit board for blood collection 1. Next, referring to FIG. 3, a description is given to the method of using the circuit board for blood collection 1.

As described above, the circuit board for blood collection 1 is used in combination with the blood-sugar-value measuring device 31 for a patient to puncture the skin of his or her finger or the like to collect blood, and measure an amount of glucose in the collected blood. To measure the amount of glucose in the blood, the patient first punctures his or her finger or the like with the puncture needle 6 to extract an extremely small amount of blood therefrom, as shown in FIG. 3(a). At this time, when the board-side stopper 8 and the insulating-side stopper 10 come to abut on skin during puncture with the puncture needle 6, further puncture is restricted thereby.

Immediately thereafter, as shown in FIG. 3(b), the electrodes 11 are brought closer into contact with a punctured portion. Then, the blood collected by puncture with the puncture needle 6 comes into contact with the surfaces of the electrodes 11 to react to the chemical agent 17. As a result, a resistance value when a voltage is applied between the individual electrodes 11 changes in accordance with an amount or value of blood sugar in the blood.
Then, as shown in FIG. 3(c), the upstream-side end portion of the circuit board for blood collection 1 in the puncture direction is inserted into a terminal input portion 32 of the blood-sugar-value measuring device 31. Consequently, the terminals 12 of the circuit board for blood collection 1 and terminals (not shown) of the terminal input portion 32 come into contact with each other. The blood-sugar-value measuring device 31 is a device for portably measuring a blood sugar value, and has the same structure as that of each of various commercially available known devices. In the blood-sugar-value measuring device 31, when the terminals 12 of the circuit board for blood collection 1 have come into contact with the terminals (not shown) of the terminal input portion 32, a predetermined voltage is applied, and the amount of glucose is measured based on the changed resistance value. The measured amount of glucose is displayed as a blood sugar value on an LED display portion 33.

In the circuit board for blood collection 1, the puncture needle 6 and the conductive pattern 4 are integrally supported on the insulating base layer 3. Therefore, it is possible to extract an extremely small amount of blood by puncture with the puncture needle 6, and easily bring the extracted blood into contact with the electrodes 11.
In the circuit board for blood collection 1, the electrodes 11, the terminals 12, and the wires 13 are integrally provided as the conductive pattern 4. Therefore, it is possible to improve the accuracy of sensing glucose in the blood in contact with the electrodes 11, and improve measurement accuracy. As a result, the circuit board for blood collection 1 allows accurate measurement of glucose in the blood with a simple structure, and allows easy operation.

In the circuit board for blood collection 1, the board-side stopper 8 and the insulating-side stopper 10 are provided on the upstream side of the puncture needle 6 in the puncture direction thereof. As a result, puncture with the puncture needle 6 is restricted by the board-side stopper 8 and the insulating-side stopper 10. This can ensure reliable and stable puncture.
In the description given above, the board-side stopper 8 and the insulating-side stopper 10 are formed in the same plan view shape, and the respective downstream-side end edges thereof in the puncture direction are disposed flush with each other.

However, as shown in FIG. 4, it is also possible to, e.g., form the board-side stopper 8 and the insulating-side stopper 10 such that the downstream-side end edge of the insulating-side stopper 10 in the puncture direction is disposed downstream in the puncture direction from the downstream-side end edge of the board-side stopper 8 in the puncture direction so as to cover the downstream-side end edge of the board-side stopper 8 in the puncture direction.
When the board-side stopper 8 and the insulating-side stopper 10 are disposed at the relative positions described above, the insulating-side stopper 10 softer than the board-side stopper 8 comes to abut on skin. As a result, it is possible to reduce pain felt by the patient upon puncture. In addition, since the board-side stopper 8 is disposed on the upstream side of the insulating-side stopper 10 in the puncture direction, the downstream-side end edge of the insulating-side stopper 10 in the puncture direction can be reinforced with the board-side stopper 8. This also allows reliable restriction of further puncture with the puncture needle 6.

In the case where puncture with the puncture needle 6 can be restricted only with the insulating-side stopper 10 by selecting the type and thickness of an insulating material, it is also possible to, e.g., form only the insulating-side stopper 10 without forming the board-side stopper 8, as shown in FIG. 5. In this case, the pain felt by the patient upon puncture can be further reduced, and trimming of the metal board 2 can be facilitated.
In the circuit board for blood collection 1, a dam 18 can also be provided around the three electrodes 11 on the surface of the insulating base layer 3, as shown in FIG. 6.

As shown in FIG. 6(a), the dam 18 is provided in continued relation on the upstream side of the three electrodes 11 in the puncture direction, on the downstream side thereof in the puncture direction, and on both widthwise sides thereof on the surface of the circuit mounting portion 9 so as to surround the three electrodes 11. The dam 18 is also formed on the surface of the insulating-side stopper 10 continuously from the surface of the circuit mounting portion 9.
In the dam 18, an insertion opening 19 is formed between the puncture needle 6 and the electrodes 11 so as to allow a straight line connecting the tip of the puncture needle 6 and the widthwise middle of the one of the electrodes 11 disposed at a most downstream position in the puncture direction to pass therethrough. By forming the insertion opening 19, it becomes easier to introduce blood collected by puncture with the puncture needle 6 into the dam 18 through the insertion opening 19, and bring the blood into contact with the three electrodes 11. The widthwise length of the insertion opening 19 is in a range of, e.g., 0.03 to 3 mm.

As shown in FIG. 6(b), the thickness of the dam 18 is the same as or larger than that of the insulating base layer 3 or the insulating cover layer 5, and is in a range of, e.g., 3 to 100 µm, or preferably 10 to 60 µm. When the thickness of the dam 18 is less than 3 µm, blood leakage may not be able to be prevented. On the other hand, when the thickness thereof is in excess of 100 µm, processing may be difficult.
Examples of a material used to form the dam 18 include a thermosetting resin such as an epoxy resin or an acrylic resin, and a thermoplastic resin such as a polycarbonate resin. It is also possible to use, apart from the synthetic resins shown above, the same synthetic resin as that of the insulating base layer 3 shown above. It is further possible to laminate a plurality of resins.

For the formation of the dam 18, there is used a method in which, e.g., a varnish of a photosensitive synthetic resin is coated on the surface of the insulating base layer 3, and cured after photoprocessing, a method in which, e.g., a film of a synthetic resin is laminated on the surface of the insulating base layer 3, an etching resist in the same pattern as that of the dam 18 is laminated on the surface of the film, and then the film exposed from the etching resist is wet-etched, a method in which e.g., a film of a synthetic resin mechanically punched in advance is laminated on the surface of the insulating base layer 3, a method in which, e.g., a film of a synthetic resin is laminated on the surface of the insulating base layer 3, and then subjected to discharge processing or laser processing, or the like. In terms of processing accuracy, the method in which a varnish of a photosensitive synthetic resin is coated on the surface of the insulating base layer 3, and then cured after photoprocessing is preferably used.

The dam 18 can also be formed continuously from and integrally with the insulating cover layer 5 simultaneously with the formation of the insulating cover layer 5.
The formation of such a dam 18 can prevent leakage of the collected blood from the electrodes 11 disposed inside the dam 18. Therefore, it is possible to achieve accurate measurement of glucose in the blood.

It is also possible to perform hydrophilic treatment, such as coating with polyvinyl pyrrolidone, with respect to the surface of the insulating base layer 3 surrounded by the dam 18.
It is further possible to provide a lid 20 covering the dam 18, as indicated by the imaginary line of FIG. 6(b). The lid has a flat-plate shape, and is formed slightly larger than the region surrounded by the dam 18. The thickness of the lid 20 is in a range of, e.g., 0.01 to 1 mm.

The lid 20 is bonded to the upper surface of the dam 18 at each of the upstream-side portion thereof in the puncture direction, the downstream-side portion thereof in the puncture direction, and the both-widthwise-side portions thereof.
As a material for forming the lid 20, the same synthetic resin as used to form the insulating base layer 3 is used. To allow easy recognition of the introduction of blood into the dam 18, a transparent synthetic resin is preferably used.

To bond the lid 20 to the upper surface of the dam 18, there is used a method in which, e.g., the dam 18 itself is formed from an adhesive material, and the lid 20 is bonded to the upper surface thereof, a method in which, e.g., an adhesive is coated onto the upper surface of the dam 18 in accordance with an ink jet method or the like, and then the lid 20 is sticked to the upper surface thereof, or the like.
By providing the lid 20, leakage of blood from the inside of the dam 18 can be prevented more reliably.

In the description given above, the board-side stopper 8 and the insulating-side stopper 10 are formed in the circuit board for blood collection 1. However, in the case where a medical expert or a patient skilled in puncture uses the circuit board for blood collection 1, it may be possible that neither of the board-side stopper 8 and the insulating-side stopper 10 needs be formed in the circuit board for blood collection 1, as shown in FIG. 7(a). In the circuit board for blood collection 1 shown in FIG. 7, neither the board-side stopper 8 nor the insulating-side stopper 10 is formed, and hence it is possible to facilitate the formation of the metal board 2 and the insulating base layer 3, and reduce cost.

In this case, it is also possible to, e.g., form the circuit mounting portion 9 of the insulating base layer 3 in the same plan view shape as that of the conductive pattern 3 or in a plan view shape similar to and slightly larger than that of the conductive pattern 3, as shown in FIG. 7(b). By thus forming the circuit mounting portion 9, it is possible to reduce an area occupied by the insulating base layer 3 that has been formed and reduce cost, while ensuring the strength of the circuit board for blood collection 1 in the longitudinal direction with the insulating base layer 3 formed along the wires 13. In FIG. 7(b), the insulating cover layer 5 is not formed, but the insulating cover layer 5 can also be formed appropriately with a required strength so as to cover the conductive pattern 4 on the surface of the insulating base layer 3.

In the description given above, the conductive pattern 4 is provided with the three electrodes 11, the three terminals 12, and the three wires 13 in correspondence to the working electrode, the counter electrode, and the reference electrode. However, as shown in FIG. 8, it is also possible to, e.g., provide the conductive pattern 4 with the two electrodes 11, the two terminals 12, and the two wires 13 in correspondence to the working electrode and the counter electrode by obviating the need for the reference electrode.
In the circuit board for blood collection 1 shown in FIG. 8, the two electrodes 11 are disposed on the downstream-side portion of the circuit mounting portion 9 in the puncture direction. These electrodes 11 are each formed in a generally rectangular plan view shape, and arranged in parallel in the puncture direction. The two electrodes 11 respectively correspond to the working electrode and the counter electrode.

The upstream-side end portion of the circuit mounting portion 9 in the puncture direction, and the circuit board portion 7 corresponding thereto are formed wide in the widthwise direction, where the two terminals 12 are arranged in parallel in the widthwise direction.
One of the two wires 13 is provided so as to extend from one widthwise side of one of the electrodes 11 toward the terminal 12 disposed on one widthwise side along the puncture direction. The other wire 13 is provided so as to extend from the other widthwise side of the other electrode 11 toward the terminal 12 disposed on the other widthwise side along the puncture direction.

In the circuit board for blood collection 1 shown in FIG. 8, the insulating cover layer 5 is not formed, and neither the board-side stopper 8 nor the insulating-side stopper 10 is formed.
In the circuit board for blood collection 1 shown in FIG. 8, the electrodes 11, the terminals 12, and the wires 13 are each smaller in number by one than those in the circuit board for blood collection 1 shown in FIG. 1 or the like. Accordingly, the circuit mounting portion 9 and the circuit board portion 7 can be formed narrower than in the circuit board for blood collection 1 shown in FIG. 1 or the like. Therefore, it is possible to reduce the size of the circuit board for blood collection 1, and simplify the structure thereof.

In the description given above, the puncture needle 6 is provided along the puncture direction. However, as shown in FIG. 9, it is also possible to, e.g., provide the puncture needle 6 in a direction crossing the longitudinal direction of the circuit board for blood collection 1, specifically along the widthwise direction.
In the circuit board for blood collection 1 shown in FIG. 9, the circuit mounting portion 9 and the circuit board portion 7 are each formed in a generally rectangular plan view shape (oblong rectangular plan view shape). The puncture needle 6 is provided so as to protrude widthwise outwardly from one widthwise end portion of the circuit board portion 7 in the vicinity of one longitudinal end portion thereof. The three electrodes 13 are disposed on one longitudinal-side portion of the circuit mounting portion 9. These electrodes 11 are each formed in a generally circular plan view shape, of which one is disposed on one widthwise side in widthwise opposed and proximate relation to the puncture needle 6. The remaining two of the electrodes 11 are disposed on the other widthwise side to be arranged in parallel on both longitudinal sides of the electrode 11 disposed on one widthwise side. Note that, in the circuit board for blood collection 1 shown in FIG. 9, the insulating cover layer 5 is not formed.

In the circuit board for blood collection 1 shown in FIG. 9, it is possible to restrict further puncture with the puncture needle 6 by one widthwise end edge of the circuit board portion 7 and the wire mounting portion 8.
In the description given above, the metal board 2, the insulating base layer 3, and the conductive pattern 4 are successively laminated in the circuit board for blood collection 1. However, as shown in FIGS. 10 and 11, it is also possible to form the conductive pattern 4 from the metal board 2 together with the puncture needle 6.

In the circuit board for blood collection 1 shown in FIG. 10, the metal board 2 integrally includes the puncture needle 6, the circuit board portion 7, and the board-side stopper 8, as shown in FIG. 10(a), and the conductive pattern 4 is disposed inside the circuit board portion 7.
The conductive pattern 4 includes the two electrodes 11, the two terminals 12, and the two wires 13. One of the electrodes 11, one of the terminals 12, and one of the wires 13 are provided continuously and integrally. The electrode 11, the terminal 12, and the wire 13 are formed by punching the circuit board portion 7 such that the respective outer peripheral edges of the electrode 11, the terminal 12, and the wire 13 are apart from the circuit board portion 7 inside the circuit board portion 7. The other electrode 11, the other terminal 12, and the other wire 13 are also provided continuously and integrally. The other electrode 11, the other terminal 12, and the other wire 13 are formed by punching the circuit board portion 7 such that the respective outer peripheral edges of the electrode 11, the terminal 12, and the wire 13 are apart from the circuit board portion 7 inside the circuit board portion 7.

The two electrodes 11 are each formed in a generally circular plan view shape, and arranged in parallel in the puncture direction.
The insulating base layer 3 is laminated on the circuit board portion 7, and formed in a pattern connecting the conductive pattern 4 disposed inside the circuit board portion 7 and the circuit board portion 7, and having openings 21 each exposing the conductive pattern 4, as shown in FIG. 10(b).

The circuit board for blood collection 1 shown in FIG. 10 is produced by preparing the metal board 2, forming the insulating base layer 3 in the foregoing pattern on the surface of the metal board 2, and then forming the conductive pattern 4 by trimming the metal board 2, while simultaneously cutting out inner portions of the circuit board portion 7 therefrom. In this manner, the circuit board for blood collection 1 can be produced in which the puncture needle 6, the circuit board portion 7, the board-side stopper 8, and the conductive pattern 4 are supported on the insulating base layer 3.

In the circuit board for blood collection 1 shown in FIG. 10, conductive pattern 4 is formed from the metal board 2 in the same layer as those of the puncture needle 6, the circuit board portion 7, and the board-side stopper 8. This can achieve a reduction in the thickness of the circuit board for blood collection 1. In addition, since the production process can be simplified, cost can be reduced.
The circuit board for blood collection 1 shown in FIG. 11 has the same structure as that of the circuit board for blood collection 1 shown in FIG. 10 except that the insulating base layer 3 is formed in a pattern in which the openings 21 for exposing the conductive pattern 4 are not formed. However, the board-side stopper 8 is not formed.

The circuit board for blood collection 1 shown in FIG. 11 is different from the circuit board for blood collection 1 shown in FIG. 10 in that the electrodes 11 and the terminals 12 are accessed from the back surface of the insulating base layer 3 in contrast to the electrodes 11 and the terminals 12 of the circuit board for blood collection 1 shown in FIG. 10, which can be accessed from the top surface of the insulating base layer 3 via the openings 21. In contrast to the circuit board for blood collection 1 shown in FIG. 10, the circuit board for blood collection 1 shown in FIG. 11 has the insulating base layer 3 which is not formed with the openings 21. Therefore, it is possible to ensure mechanical strength.

In the description given above, the circuit board for blood collection 1 has been shown as an example of the circuit board for body fluid collection of the present invention. However, the circuit board for body fluid collection of the present invention is not limited to blood collection. A target object to be collected is not particularly limited as long as it is a fluid present in a living body. For example, an intracellular fluid or an extracellular fluid can be measured as the target object. Examples of the extracellular fluid that can be listed include a blood plasma, an intercellular fluid, a lymph fluid, moistures in dense connective tissue, bone, and cartilage, and a transcellular fluid, apart from blood mentioned above.

### EXAMPLES

Hereinbelow, the present invention is described more specifically by showing the examples. However, the present invention is by no means limited to the examples.

### EXAMPLE 1 (Production of Circuit Board for Blood Collection Shown in FIG. 1)

First, a metal board made of SUS430, and having a thickness of 25 µm and a width of 350 mm was prepared (see FIG. 2(a)).

Then, on the surface of the metal board, a varnish of a photosensitive polyimide resin precursor (photosensitive polyamic acid resin) was coated, and dried by heating to form a coating. The coating was then exposed to light, and developed to be formed into a pattern. Thereafter, the coating was heated in a nitrogen atmosphere to 400 °C to form an insulating base layer having a thickness of 10 µm in a pattern having a wire mounting portion and an insulating-side stopper (see FIG. 2(b)).

Then, on the surface of the insulating base layer, a metal thin film formed of a chromium thin film having a thickness of 0.1 1 µm was formed by sputtering. Subsequently, a dry film resist was laminated on the surface of the metal thin film, exposed to light, and developed to form an etching resist in a pattern. Thereafter, the metal thin film exposed from the etching resist was wet-etched using a ferric chloride solution and a potassium ferricyanide solution as an etchant. Then, the etching resist was removed, and a conductive pattern including electrodes, terminals, and wires was formed (see FIG. 2(c)).

The thickness of the conductive pattern was 0.1 µm. The length of one side of each of the electrodes was 0. 3 mm. The length of one side of each of the terminals was 2 mm. The width of each of the wires was 25 mm.
Thereafter, on the surface of the insulating base layer, a varnish of a photosensitive polyimide resin precursor (photosensitive polyamic acid resin) was coated so as to cover the conductive pattern, and dried by heating to form a coating. The coating was then exposed to light, and developed to be formed into a pattern. Thereafter, the coating was heated in a nitrogen atmosphere to 400 °C to form an insulating cover layer having a thickness of 0.005 mm (see FIG. 2(d)). Note that the insulating cover layer was formed so as to expose the electrodes and the terminals, and cover the wires.

Then, a dry film resist was laminated on the surface of the metal board, exposed to light, and developed to form an etching resist in a pattern. Subsequently, the metal board exposed from the etching resist was etched by wet etching using ferric chloride as an etchant to be trimmed in a pattern having a puncture needle, a circuit board portion, and a board-side stopper (see FIG. 2(e)). The widthwise length of the circuit board portion was 0.3 mm. The longitudinal length of the metal board was 5 mm. The distance from the tip of the puncture needle to the nearest electrode was 0.5 mm. The angle of the tip of the puncture needle was 25°. The widthwise protruding length of the board-side stopper was 0.5 mm. The distance between the downstream-side end edge of the board-side stopper in the puncture direction and the tip of the puncture needle was 1 mm.

In this manner, a circuit board for blood collection was obtained. In the obtained circuit board for blood collection, a chemical agent containing glucose oxidase and a potassium ferricyanide solution was coated on one of the electrodes in accordance with an ink jet method (see FIG. 2(f)).

### Evaluation

A fingertip was punctured with the puncture needle, and the electrode was brought closer into contact with a blood drop squeezed out therefrom. As a result, glucose was oxidized by the blood, and ferricyanide ions reacted, so that the circuit board for blood collection was inserted into a blood-sugar-value measuring device, which allowed measurement of an amount of glucose.

During puncture with the puncture needle, the board-side stopper and the insulating-side stopper came to abut on skin, and were able to prevent the puncture needle from making a deep puncture into the skin.

### EXAMPLE 2 (Production of Circuit Board for Blood Collection (without Lid) Shown in FIG. 6)

First, a metal board made of SUS304, and having a thickness of 50 µm and a width of 350 mm was prepared (see FIG. 2(a)).

Then, on the surface of the metal board, a varnish of a photosensitive polyimide resin precursor (photosensitive polyamic acid resin) was coated, and dried by heating to form a coating. The coating was then exposed to light, and developed to be formed into a pattern. Thereafter, the coating was heated in a nitrogen atmosphere to 400 °C to form an insulating base layer having a thickness of 15 µm in a pattern having a wire mounting portion and an insulating-side stopper (see FIG. 2(b)).

Then, on the surface of the insulating base layer, a metal thin film including a nickel thin film and a chromium thin film, and having a thickness of 0.1 µm was formed by sputtering. Subsequently, a dry film resist was laminated on the surface of the metal thin film, exposed to light, and developed to form an etching resist in a pattern. Thereafter, the metal thin film exposed from the etching resist was wet-etched using a ferric chloride solution as an etchant. Then, the etching resist was removed, and a conductive pattern including electrodes, terminals, and wires was formed (see FIG. 2(c)).

The thickness of the conductive pattern was 0.1 µm. The length of one side of each of the electrodes was 200 µm. The length of one side of each of the terminals was 200 µm, The width of each of the wires was 30 mm.
Then, on the surface of the insulating base layer, a varnish of a photosensitive epoxy resin precursor was coated so as to cover the conductive pattern, and dried by heating to form a coating. The coating was then exposed to light, and developed to be formed into a pattern. Thereafter, the coating was heated in a nitrogen atmosphere to integrally form an insulating cover layer having a thickness of 50 µm and a dam (see FIG. 2(d)). Note that the insulating cover layer was formed so as to expose the electrodes and the terminals, and cover the wires.

Then, a dry film resist was laminated on the surface of the metal board, exposed to light, and developed to form an etching resist in a pattern. Subsequently, the metal board exposed from the etching resist was etched by wet etching using ferric chloride as an etchant to be trimmed in a pattern having a puncture needle, a circuit board portion, and a board-side stopper (see FIG. 2(e)). The widthwise length of the circuit board portion was 0.5 mm. The longitudinal length of the metal board was 20 mm. The distance from the tip of the puncture needle to the nearest electrode was 0.3 mm. The angle of the tip of the puncture needle was 15°. The widthwise protruding length of the board-side stopper was 0.5 mm. The distance between the downstream-side end edge of the board-side stopper in the puncture direction and the tip of the puncture needle was 0.3 mm.

In this manner, a circuit board for blood collection was obtained. In the obtained circuit board for blood collection, a chemical agent containing glucose oxidase and a potassium ferricyanide solution was coated on one of the electrodes in accordance with an ink jet method (see FIG. 2(f)).

### Evaluation

A fingertip was punctured with the puncture needle, and the electrode was brought closer into contact with a blood drop squeezed out therefrom. As a result, glucose was oxidized by the blood, and ferricyanide ions reacted, so that the circuit board for blood collection was inserted into a blood-sugar-value measuring device, which allowed measurement of an amount of glucose.

During puncture with the puncture needle, the board-side stopper and the insulating-side stopper came to abut on skin, and were able to prevent the puncture needle from making a deep puncture into the skin.

### EXAMPLE 3 (Production of Circuit Board for Blood Collection (with Lid) Shown in FIG. 6)

In the same manner as in EXAMPLE 2, a circuit board for blood collection was produced, and the dam was provided with a lid. That is, in the obtained circuit board for blood collection, a chemical agent containing glucose oxidase and a potassium ferricyanide solution was coated on one of the electrodes in accordance with an ink jet method, and then an epoxy adhesive was coated on the upper surface of the dam in accordance with an ink jet method. The lid was sticked to the upper surface, and heated at 40 °C for one hour. In this manner, the inside of the dam was covered with the lid.

The lid was formed from a polycarbonate resin into a flat-plate shape having a thickness of 75 µm, a longitudinal length of 3 mm, and a widthwise length of 2 mm.

### Evaluation

A fingertip was punctured with the puncture needle, and the electrode was brought closer into contact with a blood drop squeezed out therefrom. As a result, glucose was oxidized by the blood, and ferricyanide ions reacted, so that the circuit board for blood collection was inserted into a blood-sugar-value measuring device, which allowed measurement of an amount of glucose.

During puncture with the puncture needle, the board-side stopper and the insulating-side stopper came to abut on skin, and were able to prevent the puncture needle from making a deep puncture into the skin.

### EXAMPLE 4 (Production of Circuit Board for Blood Collection Shown in FIG. 7(a))

First, a metal board made of SUS430, and having a thickness of 100 µm and a square shape with sides each measuring 200 mm was prepared (see FIG. 2(a)).

Then, on the surface of the metal board, a varnish of a polyimide resin precursor (polyamic acid resin) was coated, and heated in a nitrogen atmosphere to 350 °C to form a polyimide film having a thickness of 2 µm. On the surface of the polyimide film, a dry film resist was laminated, exposed to light, and developed to form an etching resist in a pattern. Thereafter, the etching resist was heated at 120 °C, and the polyimide film exposed from the etching resist was wet-etched using an alkaline etchant containing potassium hydroxide and ethanolamine. Subsequently, the etching resist was removed, and an insulating base layer having a thickness of 2 µm was formed in a pattern having a wire mounting portion (see FIG. 2(b)).

Thereafter, on the surface of the insulating base layer, a metal thin film including a chromium thin film and a copper thin film was formed sequentially by sputtering. Subsequently, a dry film resist was laminated on the surface of the metal thin film, exposed to light, and developed to form a plating resist in a pattern. Then, on the surface of the metal thin film exposed from the plating resist, a plating layer made of copper was formed by electrolytic copper plating using the metal thin film as a seed film to form a conductive pattern including electrodes, terminals, and wires (see FIG. 2(c)). Thereafter, the plating resist and the metal thin film on the portion where the plating resist was formed were removed by etching.

The thickness of the conductive pattern was 10 µm. The length of one side of each of the electrodes was 0.3 mm. The length of one side of each of the terminals was 1 mm. The width of each of the wires was 0.2 mm.
Then, a film including the polyimide film having a thickness of 25 µm, and an epoxy adhesive having a thickness of 15 µm laminated on the polyimide film was mechanically punched, and the film was laminated on the surface of the insulating base layer so as to expose the electrodes and the terminals, and cover the wires (see FIG. 2(d)). Thereafter, on each of the electrodes and the terminals, a nickel plating layer having a thickness of 2 µm and a gold plating layer having a thickness of 0.5 µm were successively formed by electrolytic plating.

Subsequently, a dry film resist was laminated on the surface of the metal board, exposed to light, and developed to form an etching resist in a pattern. Then, the metal board exposed from the dry film resist was etched by wet etching using ferric chloride as an etchant to be trimmed in a pattern having a puncture needle and a circuit board portion (see FIG. 2(e)). The widthwise length of the circuit board portion was 2 mm. The longitudinal length of the metal board was 30 mm. The distance from the tip of the puncture needle to the nearest electrode was 1 mm. The angle of the tip of the puncture needle was 20°.

In this manner, a circuit board for blood collection was obtained. In the obtained circuit board for blood collection, a chemical agent containing glucose oxidase and a potassium ferricyanide solution was coated on one of the electrodes in accordance with an ink jet method (see FIG. 2(f)).

### Evaluation

A fingertip was punctured with the puncture needle, and the electrode was brought closer into contact with a blood drop squeezed out therefrom. As a result, glucose was oxidized by the blood, and ferricyanide ions reacted so that the circuit board for blood collection was inserted into a blood-sugar-value measuring device, which allowed measurement of an amount of glucose.

### EXAMPLE 5 (Production of Circuit Board for Blood Collection Shown in FIG. 9)

First, a metal board made of SUS304, and having a thickness of 50 µm and a width of 350 mm was prepared.
Then, on the surface of the metal board, a varnish of a photosensitive polyimide resin precursor (photosensitive polyamic acid resin) was coated, and dried by heating to form a coating. The coating was then exposed to light, and developed to be formed into a pattern. Thereafter, the coating was heated in a nitrogen atmosphere to 400 °C to form an insulating base layer having a thickness of 15 µm in a pattern having a wire mounting portion.

Subsequently, a dry film resist was laminated on the surface of the insulating base layer, exposed to light, and developed to form a sputtering resist in a pattern. On the surface of the insulating base layer exposed from the sputtering resist, a metal thin film made of a gold thin film and having a thickness of 0.1 µm was formed by sputtering. Thereafter, the sputtering resist was removed, and a conductive pattern including electrodes, terminals, and wires was formed.

The thickness of the conductive pattern was 0.1 µm. The length of one side of each of the electrodes was 200 µm. The length of one side of each of the terminals was 200 µm. The width of each of the wires was 30 mm.
Thereafter, a dry film resist was laminated on the surface of the metal board, exposed to light, and developed to form an etching resist in a pattern. Then, the metal board exposed from the etching resist was etched by wet etching using ferric chloride as an etchant to be trimmed in a pattern having a puncture needle and a circuit board portion. The widthwise length of the circuit board portion was 1 mm. The longitudinal length of the metal board was 15 mm. The puncture needle was provided so as to extend widthwise outwardly from one widthwise end portion of the circuit board portion 7. The length of the puncture needle was 0.5 mm. The angle of the tip of the puncture needle was 20°.

In this manner, a circuit board for blood collection was obtained. In the obtained circuit board for blood collection, a chemical agent containing glucose oxidase and a potassium ferricyanide solution was coated on one of the electrodes in accordance with an ink jet method.

### Evaluation

A fingertip was punctured with the puncture needle, and the electrode was brought closer into contact with a blood drop squeezed out therefrom. As a result, glucose was oxidized by the blood, and ferricyanide ions reacted, so that the circuit board for blood collection was inserted into a blood-sugar-value measuring device, which allowed measurement of an amount of glucose.

During puncture with the puncture needle, the widthwise end edge of the circuit board portion and the wire mounting portion came to abut on skin, and was able to prevent the puncture needle from making a deep puncture into the skin.

### EXAMPLE 6 (Production of Circuit Board for Blood Collection Shown in FIG. 10)

First, a metal board made of SUS430, and having a thickness of 20 µm and a width of 350 mm was prepared.
Then, on the surface of the metal board, a varnish of a photosensitive polyimide resin precursor (photosensitive polyamic acid resin) was coated, and dried by heating to form a coating. The coating was then exposed to light, and developed to be formed into a pattern. Thereafter, the coating was heated in a nitrogen atmosphere to 400 °C to form an insulating base layer having a thickness of 10 µm in a pattern having a wire mounting portion formed with an opening. Note that the opening was formed in a circular shape having a diameter of 300 µmφ.

Subsequently, a dry film resist was laminated on the surface of the metal board, exposed to light, and developed to form an etching resist in a pattern. Then, the metal board exposed from the etching resist was etched by wet etching using ferric chloride as an etchant to be processed in a pattern having a puncture needle, a board-side stopper, a circuit board portion, and a conductive pattern (including electrodes, terminals, and wires). The widthwise length of the circuit board portion was 0.5 mm. The longitudinal length of the metal board was 10 mm.
The distance from the tip of the puncture needle to the nearest electrode was 0.5 mm. The angle of the tip of the puncture needle was 15°.

In this manner, a circuit board for blood collection was obtained. In the obtained circuit board for blood collection, a chemical agent containing glucose oxidase and a potassium ferricyanide solution was coated on one of the electrodes from the side with the opening of the insulating base layer in accordance with an ink jet method.

### Evaluation

A fingertip was punctured with the puncture needle, and the electrode was brought closer into contact with a blood drop squeezed out therefrom. As a result, glucose was oxidized by the blood, and ferricyanide ions reacted, so that the circuit board for blood collection was inserted into a blood-sugar-value measuring device, which allowed measurement of an amount of glucose.

During puncture with the puncture needle, the board-side stopper came to abut on skin, and was able to prevent the puncture needle from making a deep puncture into the skin.

### EXAMPLE 7 (Production of Circuit Board for Blood Collection Shown in FIG. 11)

First, a metal board made of SUS304, and having a thickness of 100 µm and a square shape with sides each measuring 350 mm was prepared.

To the metal board, a polycarbonate resin film having a thickness of 100 µm and a pattern corresponding to a wire mounting portion was bonded by thermocompression to form an insulating base layer on the surface of the metal board.
Thereafter, a dry film resist was laminated on the surface of the metal board, exposed to light, and developed to form an etching resist in a pattern. Then, the metal board exposed from the etching resist was etched by wet etching using ferric chloride as an etchant to be processed in a pattern having a puncture needle, a circuit board portion, and a conductive pattern (including electrodes, terminals, and wires). The widthwise length of the circuit board portion was 3 mm. The longitudinal length of the metal board was 10 mm. The distance from the tip of the puncture needle to the nearest electrode was 0.5 mm. The angle of the tip of the puncture needle was 25°.

In this manner, a circuit board for blood collection was obtained. In the obtained circuit board for blood collection, a chemical agent containing glucose oxidase and a potassium ferricyanide solution was coated on one of the electrodes from the back side of the insulating base layer in accordance with an ink jet method.

### Evaluation

A fingertip was punctured with the puncture needle, and the electrode was brought closer into contact with a blood drop squeezed out therefrom. As a result, glucose was oxidized by the blood, and ferricyanide ions reacted, so that the circuit board for blood collection was inserted into a blood-sugar-value measuring device, which allowed measurement of an amount of glucose.

While the illustrative embodiments of the present invention are provided in the above description, such is for illustrative purpose only and it is not to be construed limitative. Modification and variation of the present invention which will be obvious to those skilled in the art is to be covered by the following claims.

### Industrial Applicability

A circuit board for body fluid collection of the present invention is connected to a device for measuring a component of a body fluid such as blood, and used to measure a component of the body fluid such as an amount of glucose in blood.

## Claims

1. A circuit board for body fluid correction comprising:
an insulating layer;
a puncture needle supported on the insulating layer; and
a conductive pattern supported on the insulating layer, and integrally including:
an electrode to be brought into contact with a body fluid collected by puncture with the puncture needle;
a terminal to be connected to a device for measuring a component of the body fluid; and
a wire electrically connecting the electrode and the terminal.

2. The circuit board for body fluid collection according to Claim 1, wherein a stopper for restricting further puncture with the puncture needle is provided on an upstream side of the puncture needle in a puncture direction thereof.

3. The circuit board for body fluid collection according to Claim 3, wherein the stopper is provided at a position spaced apart from a tip of the puncture needle by 0.3 to 2.0 mm in the puncture direction of the puncture needle.

4. The circuit board for body fluid collection according to Claim 1, wherein the insulating layer is provided with a dam that is disposed around the electrode in order to prevent leakage of the body fluid from the electrode.
